# EUROPEAN PATENT APPLICATION

(11) **EP 3 158 989 A2**
(43) Date of publication of application: **26.04.2017**
(21) Application number: 17154250.9
(22) Date of filing: 01.02.2017
(51) Int. Cl.: A61K 8/44, A61K 8/49, A61Q 19/08, A61K 8/97

(54) **COSMETIC SKIN TREATMENT COMPOSITION**

(71) Applicant: SanderStrothmann GmbH, 49124 Georgsmarienhütte (DE)
(72) Inventor: STROTHMANN, Rene, 49186 Bad Iburg (DE)
(74) Representative: f & e patent

(57) **Abstract**

The present invention refers to cosmetic compositions for skin treatment and skin care. The compositions of the present invention are intended for topical use and relate to a cosmetic composition to rejuvenate skin appearance. In particular, the present invention refers to a cosmetic skin treatment composition, comprising a) at least one plant extract comprising at least one naturally occurring antioxidant agent, and b) at least one naturally occurring amino acid comprising a second amino group and/or at least one naturally occurring amino acid comprising a hydroxyl group.

## Description

The present invention refers to cosmetic compositions for skin treatment and skin care. The compositions of the present invention are intended for topical use and relate to a cosmetic composition to rejuvenate skin appearance. In particular, the present invention refers to a cosmetic skin treatment composition, comprising a) at least one plant extract comprising at least one naturally occurring antioxidant agent, and b) at least one naturally occurring amino acid comprising a second amino group and/or at least one naturally occurring amino acid comprising a hydroxyl group.

Cosmetic compositions are widely available and are generally used for cleaning, refreshing and shaping parts of the human body (for example the skin) or for changing or decorating the appearance of the skin.

Nonetheless, the permanent use of personal care products and cosmetic compositions like e.g. soaps, shower gels, shower foams or sprays has a negative influence on the skin appearance. Furthermore, the aggressions of the environment, including climate aggressions such as exposure to sunlight, temperature variations, pollution, stress, and fatigue tend to affect the natural regeneration capacity and appearance of the skin. All of the aforementioned conditions and exposures to the skin contribute to skin ageing.

The present application aims at providing a cosmetic skin treatment composition to rejuvenate the skin appearance.

It is well recognized in the art that skin ageing is a complex multifactorial process, which cannot be reduced to a single or few factors. Over the years skin cells accumulate deleterious changes that lead to the progression of deterioration of the skin's structural integrity. These deleterious changes in the skin cells can result from the above mentioned extrinsic factors like sunlight, pollution etc., but also from intrinsic factors like mutations in the cell's genome that negatively influence the natural repair mechanism of the cell.

Skin aging includes different mechanisms, one of which is the formation of so-called advanced glycation end products (AGEs).Several of the above mentioned conditions and factors play a role in the formation of advanced glycation end products (AGEs). These AGEs are proteins or lipids that become glycated as a result of exposure to sugars.

In general, glycation is - in comparison to glycosylation, which depends on the involvement of enzymes - a non-enzymatic reaction involving a monosaccharide that reacts with an amino group of an amino acid (so-called *N*-glycation) via the Maillard reaction to form a Schiff base. The Schiff Base undergoes a molecular rearrangement, which is sometimes referred to as the Amadori rearrangement. This rearrangement may lead to crosslinking of proteins in keratinous tissue such as skin. A similar non-enzymatic reaction involving a monosaccharide and an amino acid can occur with amino acids comprising a hydroxyl group. This phenomenon of non-enzymatic formation of sugar-protein complexes increases regularly with age, and is characterized by the appearance and accumulation of AGEs.

The human skin is the largest organ in the human body and includes an outer epidermal layer and an underlying dermal layer. The epidermis is formed primarily from keratinocytes that undergo a constant renewal process as they migrate from the basal layer at the dermal/epidermal junction to the outmost layer of the stratum corneum. The dermis underlies and supports the epidermis, and is formed primarily of fibroblasts. Fibroblasts synthesize the extracellular matrix of the dermis, which includes collagen and elastin that provide the skin with strength and elasticity.

Collagen is the main structural protein in the extracellular matrix and is responsible for the strength of the dermis. In the dermis, the collagen is formed of elongated fibrils that provide the dermis with its structure and thickness. Collagen fibers are regularly renewed, but age and the above-mentioned environmental factors adversely affect collagen renewal. Moreover, in keratinous tissue like the skin the glycation reaction between monosaccharides and collagen proteins leads to the formation of early glycation products that could further develop to cross-linked collagenous structures, which also count as AGEs. Due to the crosslinks the collagen fibers decrease in elasticity. In particular, as a person ages, the body's ability to remove AGEs decreases and the rate of AGE formation outstrips the rate of collagen renewal.

A further stress factor of biological systems is the accumulation of non-usable or toxic compounds derived from biological pathways/reactions or from external intake. In healthy living organisms said non-usable or toxic compounds are transformed by enzymes called Phase I and Phase II enzymes into excretable compounds, resulting in detoxification of the cell. During aging said enzyme activity decreases, thus, the cell ballast increases.

The present application aims at providing a cosmetic skin caring and treatment composition that has excellent anti-ageing properties and that is natural and non-irritant to the skin at the same time.

A variety of skin caring agents and compositions are known in the field and thus are used in a wide range of formulations and products on the market. For example, Schmidt et al. summarize the effects of "second generation antioxidants from cress sprouts" in the International Journal for Applied Sciences (SOFW Journal, 2007). One particular focus of this review article is the role of garden cress sprout's detoxophane/sulforaphane as a cosmetic ingredient, especially its repression of Phase I enzymes like Cytochrome P450 isozyme 2E1 and its induction of Phase II enzymes like NADPH:quinine reductase 1, heme oxygenase 1 and thioredoxin reductase 1.

Furthermore, a variety of patents and patent applications discusses the use of plant extracts in cosmetic and dermatological compositions for topical use, e.g. DE102010026465A1 describes the use of extracts from soy in combination with an extract selected from the group consisting of amongst others dill, currant, cardamon, black radish, cinnamon, oat and potato in order to improve the structure of aged skin. DE102009037537A1 teaches the use of extracts from trunk, leaves or the roots of *Tinospora crispa* in combination with members of the above-mentioned group of plant extracts for the same purpose. EP2 263 643 A2 describes anti-wrinkle cosmetic products comprising plant extracts.

Antioxidant agents from citrus fruits have also been in the focus for the preparation of cosmetic compositions as they can act as protective agents against oxidative damage. Cavia-Saiz et al. describe in a comparative study the antioxidant properties, radical scavenging activity and biomolecule protection capacity of the flavonoid naringenin and its glycoside naringin *(*J Sci Food Agric. 2010 May;90(7):1238-44)*.* The effect of naringenin has been described in skin conditioning compositions, e.g. in US 5665367 (A). Cosmetic compositions employing flavonoids, in general, as antioxidants, are known. U.S. Pat. No. 5,431,912 (Nguyen et al.) discloses a cosmetic composition containing an amino acid lauroyl methionate and flavonoids (including naringenin and quercetin) to inhibit free radical formation. Nonetheless, there remains a need to provide new cosmetic skin treatment compositions that are able to improve the appearance of aged skin.

Accordingly, it was an object of the present invention to provide a cosmetic composition decreasing the skin aging effects and improving the skin appearance. Said cosmetic composition should be based on natural and/or non-irritant ingredients.

For this purpose, several ingredients were considered to be incorporated into the cosmetic skin treatment composition. It was found that a cosmetic skin treatment composition, comprising
a. at least one plant extract comprising at least one naturally occurring antioxidant agent, and
b. at least one naturally occurring amino acid comprising a second amino group and/or at least one naturally occurring amino acid comprising a hydroxyl group
results in particularly satisfying results concerning skin aging.

One of the preferred ingredients to provide skin caring properties to the cosmetic composition was at least one plant extract comprising at least one naturally occurring antioxidant agent.

In terms of the present invention, "naturally occurring antioxidant agent" refers to an antioxidant agent from plant extracts that exist by nature and that has not been artificially modified. In this connection, modification refers to any chemical modification to the antioxidant agent. Hence, the antioxidant agents used in the cosmetic skin treatment compositions of the present invention are neither artificial nor synthesized, but they are derived from a natural source.

The cosmetic composition of the present invention comprises preferably at least one plant extract deriving from a citrus fruit. Suitable citrus fruits that are preferred as source for plant extracts in the cosmetic skin treatment compositions of the present invention are grapefruit, orange, lemon, lime, or clementine. Preferably the at least one plant extract comprising at least one naturally occurring antioxidant agent derives from grapefruit. Also preferred in terms of the present invention is at least one plant extract deriving from a plant of the *Brassicaceae* family, preferably from cabbage, broccoli, cauliflower, kale, rapeseed, mustard, radishes, horse radish or garden cress, wherein an extract from garden cress is particularly preferred.

The at least one naturally occurring antioxidant agent comprised in an extract deriving from grapefruit in terms of the present invention preferably is selected from the group consisting of hesperetin and naringenin. Hesperetin is a bioflavonoid with the IUPAC name (*S*)-2,3-Dihydro-5,7-dihydroxy-2-(3-hydroxy-4-methoxyphenyl)-4*H-*1-benzopyran-4-one. It can be isolated from the paring of citrus fruits and is particularly preferred as antioxidant agent in the present invention. The other flavanone that is preferably used in the cosmetic compositions of the present invention is naringenin, which is also known as 5,7-Dihydroxy-2-(4-hydroxyphenyl)chroman-4-one under its IUPAC name. Naringenin can be isolated from grapefruit plant extracts as well. Both of these antioxidant agents independently have been described as being suitable in cosmetic compositions (e.g. US 5,665,367 and U.S. Pat. No. 5,431,912). Hesperetin and naringenin both foster the synthesis of Phase I-enzymes that support the detoxification of skin cells. Furthermore, these two antioxidant agents protect the cell's genome against UV radiation and oxidative stress. It has been shown that naringenin and hesperetin play a pivotal role in inhibiting the generation of reactive oxygen species (ROS).

The cosmetic skin treatment composition of the invention preferably includes at least one of the compounds hesperetin and naringenin, preferably both. It has been found by the inventors that the presence of hesperetin and naringenin in the cosmetic skin treatment composition is particularly effective in improving the skin appearance.

Alternatively or in addition the cosmetic skin treatment composition can comprise a plant extract from another plant, e.g. garden cress, comprising at least one further (different) naturally occurring antioxidant agent. The preferred at least one naturally occurring antioxidant agent deriving from garden cress in terms of the present invention is sulforaphane. Sulforaphane is a compound with an isothiocyanate group of organosulfur compounds and can be obtained from various vegetables such as broccoli, garden sprouts and cress or cabbages. It is also known as 1-Isothiocyanato-4-methylsulfinylbutane under its IUPAC name. Sulforaphane activates the Phase II enzymes in the skin cell and supports the resistance capacity of skin cells against environmental stress as well as against intrinsic, reactive molecules that might cause damage to the skin cell. Therefore, the presence of sulforaphane in the cosmetic skin treatment composition of the present invention is particularly preferred.

In a preferred embodiment of the invention at least two different compounds of the group hesperetin, naringenin and/or sulforaphane are incorporated in the cosmetic skin treatment composition of the present invention, preferably all three of them, hence, preferably the cosmetic skin treatment composition of the present invention comprises hesperetin, naringenin and sulforaphane as naturally occurring antioxidant agents. It has been found by the inventors that the presence of hesperetin, naringenin and sulforaphane in the cosmetic skin treatment composition is particularly effective in improving the skin appearance.

The cosmetic skin treatment composition of the present invention may therefore comprise at least two different naturally occurring antioxidant agents, preferably at least three different naturally occurring antioxidant agents. Hence, the cosmetic skin treatment composition of the present invention may comprise hesperetin and naringenin as antioxidant agents, or hesperetin and sulforaphane as antioxidant agents, or naringenin and sulforaphane as antioxidant agents, or hesperetin, naringenin and sulforaphane as antioxidant agents, preferably the cosmetic composition comprises hesperetin, naringenin and sulforaphane as antioxidant agents.

As a further ingredient the cosmetic skin treatment composition comprises at least one naturally occurring amino acid comprising a second amino group and/or at least one naturally occurring amino acid comprising a hydroxyl group. Several combinations of the ingredients of interest were considered under the focus of optimal caring properties for the cosmetic composition. It was suprisingly found that an accessory addition of at least one naturally occurring amino acid comprising a second amino group and/or at least one naturally occurring amino acid comprising a hydroxyl group into a cosmetic skin treatment composition including at least one plant extract comprising at least one naturally occurring antioxidant agent improves the effectiveness of inhibiting the progression of AGE formation in order to rejuvenate the skin appearance.

Hence, the above-mentioned object is met, according to the invention, by a cosmetic skin treatment composition, comprising
a) at least one plant extract comprising at least one naturally occurring antioxidant agent, and
b) at least one naturally occurring amino acid comprising a second amino group and/or at least one naturally occurring amino acid comprising a hydroxyl group.

In terms of the present invention, "cosmetic skin treatment composition" refers to any composition that is non-medical and non-pharmaceutical, hence, to a composition that is not intended for use in the treatment of a disease. The cosmetic compositions according to the present invention are therefore referring in particular to the treatment of the skin in terms of care without intending to treat a disease condition aiming at the healing of said disease. Hence, "cosmetic skin treatment composition" refers to a composition that has an effect on the appearance and comfort of skin without being used as a pharmaceutical or medicament.

In terms of the present invention, "naturally occurring amino acid" refers to the amino acids that are used by living organisms to synthesize their proteins. With "naturally occurring" it is meant that these amino acids have not been artificially modified, whereas modification refers to any chemical modification to the amino acid. Hence, the amino acids used in the cosmetic skin treatment compositions of the present invention are not artificial; but instead they are derived from a natural source. The preferred amino acids according to the invention are lysine, arginine, histidine, tryptophan, glutamine, asparagines or serine, threonine, tyrosine, glutamic acid and aspartic acid.

Without wishing to be bound to the theory, it is assumed that by the addition of at least one naturally occurring amino acid comprising a second amino group and/or at least one naturally occurring amino acid comprising a hydroxyl group these amino acids may function as competitors of the protein in order to intercept the sugar molecules/monosaccharides that cause the linking and crosslinking of the collagenous fibers. Hence, it is assumed that the addition of the above described amino acids protects the structural protein collagen from further crosslinking and thereby decreasing the rate of AGE formation, which results in a rejuvenated skin appearance by maintaining the skin elasticity and strength.

It was found by the inventors that a cosmetic skin caring and treatment composition could be formulated with the above described ingredients that is characterized by excellent anti-ageing properties. Furthermore, the used ingredients in the cosmetic skin treatment composition are natural and non-irritant to the skin at the same time.

In accordance with the invention, the at least one naturally occurring amino acid comprising a second amino group is preferably selected from the group consisting of lysine, arginine, histidine, and tryptophan. With regard to the at least one naturally occurring amino acid comprising a hydroxyl group, said amino acid is preferably selected from the group consisting of serine, threonine and tyrosine. Particularly preferred in the cosmetic skin treatment compositions of the present invention is lysine. It has been shown by the inventors that the addition of these amino acids to the cosmetic skin treatment compositions, in particular the addition of the amino acid lysine, is effective in improving the skin appearance upon topical use.

A particularly preferred cosmetic skin treatment composition comprises the naturally occurring antioxidant agents hesperetin, naringenin and sulforaphane as well as the naturally occurring amino acid lysine. This combination of ingredients has has been found in providing the best results with regard to improving the appearance of aged skin.

The above mentioned ingredients may be incorporated in any cosmetic composition comprising further ingredients commonly used for the preparation of cosmetic compositions. Such cosmetic compositions can be represented by e.g. sera, creams, lotions, emulsions, body milks, body oils, body sprays, gels like hydrogels, hydrodispersion gels, microemulsion gels or gelcreams, unguents, ointments and pastes or other body care products. Further, decorative cosmetic products like make-up, rouge or tanning compositions may represent cosmetic compositions according to the invention, without being limited to these. The cosmetic compositions of the present invention are preferred in form of a serum, a cream, an emulsion, or a lotion, most preferred in form of a serum. In terms of the present invention, "serum" refers to an application form that has a rapid absorption and ability to penetrate into the deeper layers of the skin. It is characterized by its non-greasy finish when applied to the skin. Sera are very high concentrated with active substances. They can be oil- and water-based.

The cosmetic composition of the present invention may be applied to mammalian keratinous tissue, in particular to human skin

Thus, the cosmetic composition preferably further comprises a dermatologically acceptable carrier wherein the at least one plant extract comprising at least one naturally occurring antioxidant agent, and the at least one naturally occurring amino acid comprising a second amino group and/or at least one naturally occurring amino acid comprising a hydroxyl group are incorporated to enable the compounds (and optional other ingredients) to be delivered to the skin. The carrier may further comprise any suitable ingredients usually used in according compositions.

The carrier may contain one or more dermatologically acceptable, hydrophilic diluents. Hydrophilic diluents include water, organic hydrophilic diluents such as lower monovalent alcohols (e.g., C1 -C4) and low molecular weight glycols and polyols, including propylene glycol, polyethylene glycol (e.g., molecular weight 200-600 g/mole), polypropylene glycol (e.g., molecular weight 425-2025 g/mole), glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof. Carriers may also be in the form of an emulsion, such as oil-in-water emulsions, water- in-oil emulsions, and water-in- silicone emulsions. An emulsion may generally be classified as having a continuous aqueous phase (e.g., oil-in-water and water-in-oil-in- water) or a continuous oil phase (e.g., water-in-oil and oil-in-water- in-oil). The oil phase may comprise silicone oils, non-silicone oils such as hydrocarbon oils, esters, ethers, and the like, and mixtures thereof. The aqueous phase may comprise water. However, the aqueous phase may also comprise components different from water, including but not limited to water-soluble moisturizing agents, conditioning agents, anti-microbials, humectants and/or other water-soluble skin care actives. Examples of a humectant are glycerin and/or other polyols. Emulsions may also contain an emulsifier. Emulsifiers may be nonionic, anionic or cationic.

Further the composition of the invention preferably may comprise skin improving minerals including calcium, magnesium, zinc, selenium or potassium. Said minerals advantageously may be provided enclosed in liposomes, allowing delivering to the cells by fusion of the liposome with the cell membrane. Said liposomes may comprise at least one of the minerals, at least two, at least three, at least four or all of the mentioned. Alternatively the composition comprises the minerals without liposomes.

Further suitable and preferred plant extracts and antioxidant agents in terms of the present invention that may be incorporated into the cosmetic compositions of the present invention are:
▪ ascorbic acid (vitamin C) isolated from citrus fruits, billygoat plum (Terminalia ferdinandiana), or camu camu (Myrciaria dubia);
▪ tocopherol (vitamin E) isolated from wheat germ oil, sunflower oil, red palm oil, or olive oil;
▪ polyphenolic antioxidant agents like resveratrol isolated from grapes, raspberry, or mulberry; like flavonoides isolated from arnica (Arnica montana), birch (Betula pubescens or Betula pendula), buckwheat (Fagopyrum esculentum), ginkgo (Ginkgo biloba), European goldenrod (Solidago virgaurea, Solidago gigantea, Solidago canadensis), elderberry (Sambucus nigra), common hop (Humulus lupulus), camomile (Matricaria chamomilla L.), Sibirian Larch (Larix sibirica), meadowsweet (Filipendula ulmaria), cardus marianus (Silybum marianum), purple passionflower (Passiflora incarnata), pot marigold (Calendula officinalis), roman chamomile (Chamaemelum nobile), common grape vine (Vitis vinifera), safflower (Carthamus tinctorius), pansy (Viola arvensis, Viola tricolor), licorice (Glycyrrhiza glabra), or hawthorn (Crataegus) ; and like carnosic acid isolated from rosemary (Rosmarinus officinalis), common sage (Salvia officinalis)
▪ carotenoids like lycopene isolated from tomato or water melon; like betacarotenes isolated from carrots, sweet potatoes, pumpkins, kaki, apricots, papayas, mangos, nectarines, peaches, pears, sallow thorn, spinach, broccoli, endives, chicory, cress, beetroot, or dandelion; like lutein isolated form spinach, or green cabbage.

Further optional components/ingredients are listed below:
A wide variety of optional components/ingredients may be included in the cosmetic compositions. For example, the cosmetic compositions may include absorbents, abrasives, anti-caking agents, antifoaming agents, antimicrobial agents, binders, biological additives, buffering agents, bulking agents, chemical additives, cosmetic biocides, denaturants, cosmetic astringents, drug astringents, external analgesics, film formers, humectants, opacifying agents, fragrances, pigments, colourings, essential oils, skin sensates, emollients, skin soothing agents, skin healing agents, pH adjusters, plasticizers, preservatives, preservative enhancers, propellants, reducing agents, additional skin-conditioning agents, skin penetration enhancing agents, skin protectants, solvents, suspending agents, emulsifiers, thickening agents, solubilizing agents, sunscreens, sunblocks, ultraviolet light absorbers or scattering agents, sunless tanning agents, further antioxidants and/or radical scavengers, chelating agents, sequestrants, anti-acne agents, antiinflammatory agents, anti-androgens, depilation agents, desquamation agents/exfoliants, organic hydroxy acids, vitamins and derivatives thereof, and other natural extracts. Such other materials are known in the art. Nonexclusive examples of such materials are described in Harry's Cosmeticology, 7th Ed., Harry and Wilkinson (Hill Publishers, London 1982); in Pharmaceutical Dosage Forms- Disperse Systems; Lieberman, Rieger and Banker, Vols. 1 (1988) and 2 (1989); Marcel Decker, Inc.; in The Chemistry and Manufacture of Cosmetics, 2nd. Ed., deNavarre (Van Nostrand 1962-1965); and in The Handbook of Cosmetic Science and Technology, 1 st Ed.. Knowlton and Pearce (Elsevier 1993). In a particularly preferred embodiment of the invention the cosmetic composition of the present invention is free from cortisone or any cortisone derivative.

All the ingredients suitable for the particular composition type are known to persons skilled in the art. The following examples of ingredients may be comprised in the compositions of the invention:

### Oil/Oil bodies

A cream, lotion or body milk comprises typically oily and/or fatty components.Suitable oil bodies are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl be- henate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈-alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆ -Ci₀-fatty acids, liquid mono-/di-/triglyceride mixtures based on C₆-C₁₈-fatty acids, esters of C₆- C₂₂- fatty alcohols and/or Guerbet alcohols with aromatic carboxylic acids, in particular benzoic acid, esters of C₂- C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol^{(R)} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{(R)} TN), linear or branched, symmetrical or asymmetrical dialkyl ethers having 6 to 22 carbon atoms per alkyl group, such as, for example, dicaprylyl ether (Cetiol^{(R)} OE), ring-opening products of epox- idized fatty acid esters with polyols, silicone oils (cyclomethicones, silicone methicone grades, etc.), aliphatic or naphthenic hydrocarbons, such as, for example, squalane, squa- lene or dialkylcyclohexanes, and/or mineral oils.

### Waxes

Among the group of suitable waxes one can differentiate between the following types:
▪ superfatting agents
▪ consistency factors
▪ pearlising waxes, and
▪ natural waxes

Superfatting agents may be selected from substances such as for example, lanolin and lecithin and also polyethoxylated or acylated lanolin and lecithin derivatives, polyol fatty acid esters, monoglycerides and fatty acid alkanolamides; fatty acid alkanolamides can also serve as foam stabilizers.

Consistency factors can be for example fatty alcohols or hydroxyfatty alcohols containing 12 to 22 and preferably 16 to 18 carbon atoms and also partial glycerides, fatty acids or hydroxyfatty acids of the same carbon range. A combination of these substances with alkyl oligoglucosides and/or fatty acid N-methyl glucamides of the same chain length and/or polyglycerol poly-12-hydroxystea rates is preferably used.

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

Besides natural oils used, natural waxes may also be present in the compositions, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, monta n ester waxes, sasol waxes, hydrogenated jojoba waxes and synthetic waxes such as, for example, polyalkylene waxes and polyethylene glycol waxes.

### Emulsifiers

The emulsifiers, detergents or surfactants may be of non-ionic, anionic, cationic and/or amphoteric nature. In particular preferred are non-ionic emulsifiers, such as:
▪ products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
▪ 12/18 fatty acid monoesters and diesters of addition products of 1 to 30 mol ethylene oxide onto glycerol;
▪ glycerol mono- and diesters and sorbitan mono- and diesters of saturated and unsaturated fatty acids containing 6 to 22 carbon atoms and ethylene oxide addition products thereof;
▪ addition products of 15 to 60 mol ethylene oxide onto castor oil and/or hydrogenated castor oil;
▪ polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
▪ addition products of 2 to 15 mol ethylene oxide onto castor oil and/or hydrogenated castor oil;
▪ partial esters based on linear, branched, unsaturated or saturated C₆/₂₂ fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
▪ mono-, di and trialkyl phosphates and mono-, di- and/or tri-PEG-alkyl phosphates and salts thereof;
▪ wool wax alcohols;
▪ polysiloxane/polyalkyl polyether copolymers and corresponding derivatives;
▪ mixed esters of pentaerythritol, fatty acids, citric acid and fatty alcohol and/or mixed esters of C₆-₂₂ fatty acids, methyl glucose and polyols, preferably glycerol or polyglycerol,
▪ polyalkylene glycols and
▪ glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/16} fatty acid monoesters and diesters of addition products of ethylene oxide onto glycerol are known as lipid layer enhancers for cosmetic formulations. The most preferred emulsifiers are described in more detail as follows:
Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

Typical examples of suitable polyglycerol esters are Polyglyceryl-2 Dipolyhydroxystearate (Dehymuls PGPH), Polyglycerin-3-Diisostearate (Lameform TGI), Polyglyceryl-4 Isostearate (Isolan GI 34), Polyglyceryl-3 Oleate, Diisostearoyl Polyglyceryl-3 Diisostearate (Isolan^{(R)} PDI), Polyglyceryl-3 Methylglucose Distearate (Tego Care 450), Polyglyceryl-3 Beeswax (Cera Bellina), Polyglyceryl-4 Caprate (Polyglycerol Caprate T2010/90), Polyglyceryl-3 Cetyl Ether (Chimexane N L), Polyglyceryl-3 Distearate (Cremophor^{(R)} GS 32) and Polyglyceryl Polyricinoleate (Admul WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Typical anionic emulsifiers are aliphatic C12-22 fatty acids, such as palmitic acid, stearic acid or behenic acid for example, and C12-22 dicarboxylic acids, such as azelaic acid or sebacic acid for example.

Other suitable emulsifiers are amphoteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl- 3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of Cocamidopropyl Betaine is particularly preferred. Ampholytic surfactants are also suitable emulsifiers. Am pholytic surfactants are surface-active compounds which, in addition to a C₈/₁₈ alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N- alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### Active principles

The compositions according to the present invention may contain additional ingredients encompassed by the term "active principles". Examples for suitable ingredients are abrasives, anti-acne agents, additional agents against skin ageing, anti-cellulitis agents, antidandruff agents, anti-inflammatory agents, irritation-preventing agents, irritation-inhibiting agents, further antioxidants, astringents, perspiration-inhibiting agents, antiseptic agents, anti-statics, binders, buffers, carrier materials, chelating agents, cell stimulants, cleansing agents, care agents, depilatory agents, surface-active substances, deodorizing agents, softeners, enzymes, essential oils, fibres, film-forming agentsgelling agents, gel-forming agents, moisture-donating agents, moisturizing substances, moisture-retaining substances, bleaching agents, strengthening agents, stain-removing agents, optically brightening agents, dirt-repellent agents, friction-reducing agents, lubricants, opacifying agents, covering agents, gloss agents, polymers, powders, proteins, re-oiling agents, abrading agents, silicones, skin-soothing agents, skin-cleansing agents, skin care agents, skin-healing agents, skin-lightening agents, skin-protecting agents, skin-softening agentsskin-cooling agents, skin-warming agents, stabilizers, UV- absorbing agents, UV filters, detergents, suspending agents, skin- tanning agents, thickeners, vitamins, oils, waxes, fats, phospholipids, saturated fatty acids, mono- or polyunsaturated fatty acids, hydroxy acids, polyhydroxyfatty acids, liquefiers, dyestuffs, pigments, anti-corrosives, odoriferous substances, polyols, surfactants, electrolytes, organic solvents or silicone derivatives and the like as additional auxiliaries and additives.

Examples for further ingredients suitable for incorporation into the skin treatment composition of the present invention can be found in EP 3 090 726 A in paragraphs [0051] to [0093], herein incorporated by reference.

Out of the above-mentioned and suitable further ingredients, it is particularly preferred that the cosmetic composition of the present invention besides of the ingredients described above as preferred further comprises at least one of the ingredients selected from the group consisting of water, sorbitol, glycerin, urea, hyaluronic acid and liposomes, wherein it is preferred that said liposomes comprise minerals selected from calcium, magnesium, zinc, selenium or potassium.

It has been shown that the addition at least one of the ingredients selected from the group consisting of water, sorbitol, glycerin, urea, liposomes, and hyaluronic acid to the cosmetic skin treatment composition of the present invention further improves the appearance of the skin.

In case the cosmetic skin treatment composition of the present invention comprises sorbitol, this ingredient is present in amounts of 0.1 to 10 wt.-% of the total weight of the composition, preferably in amounts of 0.2 to 8 wt.-% of the total weight of the composition, more preferred in amounts of 0.5 to 5 wt.-% of the total weight of the composition, even more preferred in amounts of 0.75 to 4 wt.-% of the total weight of the composition, even more preferred in amounts of 2 to 3 wt.-% of the total weight of the composition, and most preferred in amounts of 2.5 wt.-% of the total weight of the composition.

In case the cosmetic skin treatment composition of the present invention comprises glycerin, this ingredient is present in amounts of 0.1 to 25 wt.-% of the total weight of the composition, preferably in amounts of 0.2 to 20 wt.-% of the total weight of the composition, more preferred in amounts of 0.5 to 15 wt.-% of the total weight of the composition, even more preferred in amounts of 1 to 10 wt.-% of the total weight of the composition, even more preferred in amounts of 2.5 to 7.5 wt.-% of the total weight of the composition, and most preferred in amounts of 5 wt.-% of the total weight of the composition.

In case the cosmetic skin treatment composition of the present invention comprises urea, this ingredient is present in amounts of 0.001 to 0.1 wt.-% of the total weight of the composition, more preferred in amounts of 0.005 to 0.05 wt.-% of the total weight of the composition, and most preferred in amounts of 0.01 wt.-% of the total weight of the composition.

In case the cosmetic skin treatment composition of the present invention comprises liposomes, this ingredient is present in amounts of 0.01 to 10 wt.-% of the total weight of the composition, preferably in amounts of 0.05 to 7 wt.-% of the total weight of the composition, more preferred in amounts of 0.1 to 5 wt.-% of the total weight of the composition, even more preferred in amounts of 0.5 to 2 wt.-% of the total weight of the composition, and most preferred in amounts of 1 wt.-% of the total weight of the composition. As mentioned above, it is particularly preferred that the liposomes comprise minerals selected from calcium, magnesium, zinc, selenium or potassium.

In case the cosmetic skin treatment composition of the present invention comprises hyaluronic acid, this ingredient is present in amounts of 0.01 to 0.1 wt.-% of the total weight of the composition, more preferred in amounts of 0.02 to 0.08 wt.-% of the total weight of the composition, and most preferred in amounts of 0.05 wt.-% of the total weight of the composition.

According to the present invention each of the plant extracts comprising at least one naturally occurring antioxidant agent is present in amounts of 0.01 to 5 wt.-% of the total weight of the composition, preferably in amounts of 0.05 to 3 wt.-% of the total weight of the composition, more preferred in amounts of 0.1 to 2 wt.-% of the total weight of the composition, even more preferred in amounts of 0.2 to 0.8 wt.-% of the total weight of the composition, even more preferred in amounts of 0.4 to 0.6 wt.-% of the total weight of the composition, and most preferred in amounts of 0.5% wt.-% of the total weight of the composition. It is preferred that each of the plant extracts comprises any of the naturally occurring antioxidant agent in an amount of 10% to 100%. Thus, the naturally occurring antioxidant agent is present in amounts of 0.001 to 5 wt.-% of the total weight of the composition, preferably in amounts of 0.005 to 3 wt.-% of the total weight of the composition, more preferred in amounts of 0.01 to 2 wt.-% of the total weight of the composition, even more preferred in amounts of 0.02 to 0.8 wt.-% of the total weight of the composition, even more preferred in amounts of 0.04 to 0.6 wt.-% of the total weight of the composition, and most preferred in amounts of 0.05 to 0.5% wt.-% of the total weight of the composition

According to the present invention each of the at least one naturally occurring amino acid comprising a second amino group and/or at least one naturally occurring amino acid comprising a hydroxyl group are present in amounts of 0.01 to 5 wt.-% of the total weight of the composition, preferably in amounts of 0.02 to 2 wt.-% of the total weight of the composition, more preferred in amounts of 0.05 to 1.5 wt.-% of the total weight of the composition, and most preferred in amounts of 0.1 wt.-% of the total weight of the composition.

It is preferred that the cosmetic skin treatment composition of the present invention comprises a) at least one plant extract comprising at least one naturally occurring antioxidant agent, and b) at least one naturally occurring amino acid comprising a second amino group and/or at least one naturally occurring amino acid comprising a hydroxyl group, wherein the weight ratio between a) and b) is in the range of 100:1 to 1:10, preferably in a weight ratio of 50:1 to 1:5, more preferred in a weight ratio of 20:1 to 1:1, even more preferred in a weight ratio of 10:1 to 1:1, and most preferred in a weight ratio of 5:1. The aforementioned weight ratios refer to the sum of the present plant extracts in the composition comprising the at least one naturally occurring antioxidant agent and the at least one naturally occurring amino acid comprising a second amino group and/or at least one naturally occurring amino acid comprising a hydroxyl group, respectively.

As mentioned above, it is preferred that the cosmetic skin treatment composition of the present invention comprises hesperetin, naringenin and sulforaphane as naturally occurring antioxidant agents. When these three naturally occurring antioxidant agents are present in the same cosmetic composition, they are present in a weight ratio of 10:10:1: to 10:1:10 to 1:10:10 for hesperetin, naringenin and sulforaphane, preferably in a weight ratio of 5:5:1 to 5:1:5 to 1:5:5, more preferred in a weight ratio of 2:2:1 to 2:1:2 to 1:2:2, most preferred in a weight ratio of 1:1:1 for hesperetin, naringenin and sulforaphane, respectively.

It is further preferred that the cosmetic skin treatment composition comprises besides the components hesperetin, naringenin and sulforaphane further an amino acid comprising a further amino group, preferably the amino acid lysine. This particular combination of the ingredients results in an outstanding positive effect on anti-aging of the skin, in particular to the decrease of the formation of AGEs.

It has been found that the ingredients are particularly effective when they are added in amounts of the above mentioned ranges in a cosmetic composition.

The composition of the present invention can be formulated in any form that is applicable to the body, in particular to the skin. For this purpose the compositions of the present invention can be formulated e.g. in form of a serum, a cream, an emulsion, or a lotion, preferably in form of a serum.

In particular, the cosmetic compositions of the present invention are for topical use, especially for the care of aged skin, wherein the ageing might be caused by the glycation of proteins, especially collagen fibers, with monosaccharides to form advanced glycation end products (AGEs).

## Claims

1. A cosmetic skin treatment composition, comprising
a. at least one plant extract comprising at least one naturally occurring antioxidant agent, and
b. at least one naturally occurring amino acid comprising a second amino group and/or at least one naturally occurring amino acid comprising a hydroxyl group.

2. The cosmetic composition according to claim 1, wherein the composition further comprises at least one of the following ingredients selected from the group consisting of water, sorbitol, glycerine, urea, hyaluronic acid and liposomes, wherein said liposomes preferably comprises minerals selected from minerals comprising calcium, magnesium, zinc, selenium or potassium,

3. The cosmetic composition of any of the preceding claims, wherein the at least one plant extract derives from a citrus fruit, preferably from grapefruit, orange, lemon, lime, or clementine, more preferred from grapefruit, and/or the at least one plant extract derives from a plant of the *Brassicaceae* family, preferably from cabbage, broccoli, cauliflower, kale, rapeseed, mustard, radishes, horse radish or garden cress, preferably from garden cress.

4. The cosmetic composition according to claim 3, wherein the at least one naturally occurring antioxidant agent deriving from grapefruit is selected from the group consisting of hesperetin and naringenin, and/or the at least one naturally occurring antioxidant agent deriving from garden cress is sulforaphane.

5. The cosmetic composition of claim 4, wherein the composition comprises at least two different naturally occurring antioxidant agents, preferably at least three different naturally occurring antioxidant agents.

6. The cosmetic composition according to claim 5, wherein the cosmetic composition comprises hesperetin and naringenin as antioxidant agents, or wherein the cosmetic composition comprises hesperetin and sulforaphane as antioxidant agents, or wherein the cosmetic composition comprises naringenin and sulforaphane as antioxidant agents, or wherein the cosmetic composition comprises hesperetin, naringenin and sulforaphane as antioxidant agents, wherein preferably the cosmetic composition comprises hesperetin, naringenin and sulforaphane as antioxidant agents.

7. The cosmetic composition according to any of the preceding claims, wherein the at least one naturally occurring amino acid comprising a second amino group is selected from the group consisting of lysine, arginine, histidine, and tryptophan, preferably the at least one naturally occurring amino acid comprising a second amino group is lysine.

8. The cosmetic composition according to any of the preceding claims, wherein the at least one naturally occurring amino acid comprising a hydroxyl group is selected from the group consisting of serine, threonine and tyrosine.

9. The cosmetic composition according to any of the preceding claims, wherein each of the at least one extract comprising any naturally occurring antioxidant agent is present in amounts of 0.01 to 5 wt.-% of the total weight of the composition, preferably in amounts of 0.05 to 3 wt.-% of the total weight of the composition, more preferred in amounts of 0.1 to 2 wt.-% of the total weight of the composition, even more preferred in amounts of 0.2 to 0.8 wt.-%, even more preferred in amounts of 0.4 to 0.6 wt.-% of the total weight of the composition, and most preferred in amounts of 0.5% wt.-% of the total weight of the composition.

10. The cosmetic composition according to any of the preceding claims, wherein each of the at least one naturally occurring amino acid comprising a second amino group and/or at least one naturally occurring amino acid comprising a hydroxyl group is present in amounts of 0.01 to 5 wt.-% of the total weight of the composition, preferably in amounts of 0.02 to 2 wt.-% of the total weight of the composition, more preferred in amounts of 0.05 to 1.5 wt.-% of the total weight of the composition, and most preferred in amounts of 0.1 wt.-% of the total weight of the composition.

11. The composition of any of the preceding claims, further comprising at least one mineral comprising calcium, magnesium, zinc, selenium or potassium, preferably two, more preferred three, even more preferred four and most preferred all of them.

12. The composition of any of the preceding claims in form of a serum, a cream, an emulsion, or a lotion, preferably in form of a serum.

13. The composition of any of the preceding claims for topical use.

14. Use of a lysine in a cosmetic composition for decreasing the formation of glycolation of proteins.
